(19) **Europäisches Patentamt / European Patent Office / Office européen des brevets**

(11) **EP 3 362 487 B1**

(12) **FASCICULE DE BREVET EUROPEEN**

(45) Date de publication et mention de la délivrance du brevet:
**27.07.2022 Bulletin 2022/30**

(21) Numéro de dépôt: **16794381.0**

(22) Date de dépôt: **14.10.2016**

(51) Classification Internationale des Brevets (IPC):
**C08B 37/16** (2006.01)     **A61K 47/40** (2006.01)
**C08L 5/16** (2006.01)

(52) Classification Coopérative des Brevets (CPC):
**C08B 37/0012; A61K 31/4965; A61K 31/724;
A61K 47/40; C08B 37/0015; C08L 5/16;**
C08L 2203/02

(86) Numéro de dépôt international:
**PCT/FR2016/052658**

(87) Numéro de publication internationale:
**WO 2017/064436 (20.04.2017 Gazette 2017/16)**

(54) **NOUVELLES CYCLODEXTRINES MÉTHYLÉES ET LEURS PROCÉDÉS DE PRÉPARATION**

NEUARTIGE METHYLIERTE CYCLODEXTRINE UND VERFAHREN ZUR HERSTELLUNG DAVON

NOVEL METHYLATED CYCLODEXTRINS AND METHODS FOR THE PRODUCTION THEREOF

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **16.10.2015 FR 1559832**

(43) Date de publication de la demande:
**22.08.2018 Bulletin 2018/34**

(73) Titulaire: **Roquette Frères**
**62136 Lestrem (FR)**

(72) Inventeurs:
• **PARISSAUX, Xavier**
 **62120 Campagnes Lez Wardrecques (FR)**
• **PALMIERI, Jean-Baptiste**
 **59160 Lomme (FR)**
• **IBERT, Mathias**
 **59930 La Chapelle d'Armentieres (FR)**
• **BUFFE, Clothilde**
 **59160 Lomme (FR)**

(74) Mandataire: **Plasseraud IP**
 **66, rue de la Chaussée d'Antin**
 **75440 Paris Cedex 09 (FR)**

(56) Documents cités:
EP-A2- 0 193 850    WO-A1-94/02516
WO-A1-94/22461    CN-A- 1 709 918
JP-A- S62 220 501    US-A- 5 710 268

• M. PUMERA ET AL: "Determination of cyclodextrins and their derivatives by capillary electrophoresis with indirect UV and conductivity detection", FRESENIUS JOURNAL OF ANALYTICAL CHEMISTRY., vol. 369, no. 7-8, 24 avril 2001 (2001-04-24), pages 666-669, XP055269412, DE ISSN: 0937-0633, DOI: 10.1007/s002160100721

## Description

La présente invention se rapporte à une nouvelle cyclodextrine méthylée, ainsi qu'à un nouveau procédé utile à sa préparation. L'invention se rapporte également à l'utilisation de cette cyclodextrine méthylée, pour la solubilisation de composés lipophiles ou porteurs d'au moins un groupement lipophile. L'invention concerne également une composition comprenant cette cyclodextrine méthylée, notamment une composition pharmaceutique, en particulier destinée à être utilisée comme médicament, et plus particulièrement pour son utilisation dans le traitement et/ou la prévention du diabète de type 2 et/ou de ses complications, et/ou de maladies susceptibles d'être traitées et/ou prévenues par une augmentation du taux de cholestérol-HDL et/ou par une réduction ou prévention des plaques d'athérome, et/ou de maladies du système nerveux central..

## Contexte de l'Invention

Les cyclodextrines sont des oligosaccharides cycliques provenant de la dégradation enzymatique de l'amidon. Les trois cyclodextrines naturelles les plus courantes se composent de 6, 7 ou 8 unités $\alpha$-D-glucopyranose en configuration chaise reliées entre elles par des liaisons $\alpha$-1,4. On les appelle plus couramment $\alpha$, $\beta$, ou $\gamma$ cyclodextrine, respectivement. Leur structure en trois dimensions apparaît sous la forme d'un cône tronqué à l'extérieur duquel se trouvent les groupements hydroxyles représentant la partie hautement hydrophile des cyclodextrines. L'intérieur du cône ou la cavité des cyclodextrines est constitué par les atomes d'hydrogène portés par les carbones $C_3$ et $C_5$ ainsi que par les atomes d'oxygène participant à la liaison glycosidique, leur conférant ainsi un caractère apolaire.

Les cyclodextrines présentant une partie extérieure hydrophile et une cavité hydrophobe sont généralement utilisées pour leur capacité à encapsuler les composés ou groupements lipophiles, et donc, pour leur rôle de protecteur et de solubilisant de ces composés lipophiles ou porteurs de groupements lipophiles. On les retrouve ainsi classiquement dans les domaines de l'agroalimentaire, mais aussi en galénique où elles sont utilisées comme excipient dans des formulations pharmaceutiques.

Les unités glucopyranoses des cyclodextrines comprennent chacune 3 groupements hydroxyles réactifs, qui sont portés par les carbones C2, C3 et C6. De nombreux dérivés ont ainsi déjà été synthétisés par greffage de différents groupements sur ces fonctions hydroxyles, parmi lesquels on peut citer les hydroxypropyl-cyclodextrines, les méthyl-cyclodextrines, et les dérivés sulfatés.

Très récemment, dans sa demande de brevet WO 2015/087016 A1, la Demanderesse a démontré de façon originale que certaines méthyl-cyclodextrines, caractérisées par un faible degré de substitution molaire (MS), compris entre 0,05 et 1,50, pouvaient être utilisées non pas en tant qu'excipient, mais en tant que principe actif pharmaceutique.

La Demanderesse a montré en particulier que ces méthyl-cyclodextrines de faible MS étaient capables d'agir efficacement et spécifiquement sur le métabolisme lipidique, en augmentant le taux de cholestérol-HDL plasmatique (« bon cholestérol »), et en diminuant la triglycéridémie.

Dans cette demande de brevet, la Demanderesse envisageait la possibilité d'une forme galénique qui permette de tirer avantage à la fois des propriétés pharmacologiques de ces méthyl-cyclodextrines, mais également de leurs propriétés d'encapsulation. Il s'agissait d'envisager en particulier, un complexe dans lequel cette méthyl-cyclodextrine aurait encapsulé un principe actif hydrophobe ayant une activité pharmacologique complémentaire.

Malheureusement le produit utilisé dans cette demande de brevet possède une capacité à solubiliser les composés lipophiles relativement limitée.

Le principal objectif de la présente invention était donc d'améliorer cette capacité de solubilisation de la méthyl-cyclodextrine, afin d'obtenir un actif / excipient de plus grand efficacité.

La contrainte majeure à laquelle la Demanderesse a dû faire face est que, conformément à ce qui était enseigné dans la demande de brevet WO 2015/087016 A1 précitée, ces méthyl-cyclodextrines devaient impérativement présenter un faible MS pour pouvoir agir efficacement sur le métabolisme lipidique. Or il est connu que les cyclodextrines faiblement méthylées possèdent une plus faible capacité à solubiliser les composés lipophiles, comparativement au cyclodextrines hautement méthylées (à ce sujet par exemple : Kiss, T., Fenyvesi, F. et al.: Eur. J. Pharm. Sci. 40 (2010) 376-380).

Aussi afin d'obtenir une méthyl-cyclodextrine qui soit à la fois active sur le plan pharmacologique, et à la fois capable d'encapsuler plus efficacement des composés lipophiles, il fallait pouvoir concilier des propriétés *a priori* contradictoires.

## Présentation de l'invention

La Demanderesse y est parvenue après de nombreuses études qui se sont soldées par la mise au point d'une cyclodextrine méthylée de faible MS, compris entre 0,05 et 1,50, qui, à la différence du produit utilisé dans cette précédente demande de brevet, présente une faible conductivité lorsqu'elle est sous la forme d'une solution aqueuse, inférieure ou égale à 50 $\mu$S/cm.

La méthyl-cyclodextrine de l'invention a pu être obtenue selon un nouveau procédé, faisant intervenir une étape de diminution des espèces ioniques de manière à ce que la conductivité de la méthyl-cyclodextrine soit fortement réduite.

L'utilisation de méthyl-cyclodextrines comme solubilisant de principes actifs lipophiles avait bien sûr déjà été envisagé dans l'art antérieur. Cependant ces méthyl-cyclodextrines ne présentaient pas les caractéristiques et donc l'efficacité de celles de l'invention.

Le brevet US 7,259,153 B2 par exemple décrit la solubilisation de principe actifs lipophiles à l'aide de méthyl-cyclodextrines, en particulier de méthyl-$\beta$-cyclodextrines (M$\beta$CD). Cependant ces M$\beta$CD ne présentaient pas la combinaison de caractéristiques des méthyl-cyclodextrines de l'invention et sont bien moins efficaces pour la solubilisation de composés lipophiles (voir en particulier les résultats obtenus avec la méthyl-cyclodextrine comparative « M$\beta$CD-CP1 » dans l'Exemple ci-après).

Notons que dans ce brevet, le problème de la solubilisation était en principe résolu au moyen de l'utilisation de M$\beta$CD cristallisées. Le fait que ces cyclodextrines requièrent d'être sous forme cristalline limite considérablement la liberté de texturation du produit.

D'autres documents décrivent de façon plus générale des procédés de préparation de cyclodextrines méthylées. Parmi les rares documents décrivant effectivement la préparation de méthyl-cyclodextrines de faibles MS, la Demanderesse a constaté encore que les procédés employés ne permettaient pas de séparer efficacement les espèces ioniques et donc d'aboutir à l'obtention d'un produit de faible conductivité conformément à l'invention.

Le brevet US 6,602,860 B1 par exemple, détenu par la Demanderesse, décrit des compositions comprenant des méthyl-cyclodextrines ayant un MS inférieur à 2. Cependant, le procédé employé ne permettait pas la préparation d'un produit de faible conductivité conformément à l'invention. Ces M$\beta$CD sont bien moins efficaces que les méthyl-cyclodextrines de l'invention pour la solubilisation de composés lipophiles. (voir en particulier les résultats obtenus avec la méthyl-cyclodextrine comparative « M$\beta$CD-CP2 » dans l'Exemple ci-après).

De plus, ces méthyl-cyclodextrines, étaient caractérisées par le fait qu'elles se présentaient à la fois sous forme cristalline et sous forme amorphe, or il est difficile industriellement de fournir un produit qui présente toujours le même rapport cristallin / amorphe. De plus, la portion cristalline conduit, en présence de faibles taux d'humidité, à la cristallisation de la portion initialement amorphe. Cette cristallisation conduit à une poudre qui présente des problèmes de mottage. En plus de problème de reproductibilité du procédé, on obtient donc un produit relativement instable.

Ainsi, la présente invention a pour objectif de fournir une cyclodextrine méthylée de faible MS, qui soit plus efficace que les cyclodextrine méthylées de faible MS de l'art antérieur, pour la solubilisation de composés lipophiles ou porteurs d'au moins un groupement lipophile.

Plus généralement, la présente invention a pour objectif de fournir un moyen de solubilisation en milieu aqueux de composés lipophiles, ou porteurs d'au moins un groupement lipophile.

La présente invention a en particulier pour objectif de fournir un moyen de solubilisation en milieu aqueux de composés lipophiles, ou porteurs d'au moins un groupement lipophile, qui permette en outre d'agir positivement sur le métabolisme lipidique, notamment en augmentant le taux sérique de cholestérol-HDL et/ou en diminuant la triglycéridémie.

La présente invention a en outre pour objectif de fournir une cyclodextrine utile pour améliorer la stabilité chimique de composés lipophiles ou porteurs d'au moins un groupement lipophile, et/ou pour améliorer leur délivrance au niveau et à travers des membranes biologiques, et/ou pour augmenter leur stabilité physique, et/ou pour les convertir d'une forme liquide à une forme pulvérulente, et/ou pour prévenir les interactions avec d'autres composés, et/ou pour réduire l'irritation locale après une administration topique ou orale de ces composés lipophiles ou porteurs de groupements lipophiles, et/ou pour prévenir leur absorption au niveau des certains tissus comme la peau, et/ou pour obtenir une libération prolongé de ces composés, et/ou pour masque leur goût, en particulier leur amertume, et/ou pour modifier leur biodisponibilité.

## Résumé de l'invention

La présente invention a ainsi pour premier objet une méthyl-cyclodextrine présentant un degré de substitution molaire (MS) compris entre 0,05 et 1,50, caractérisée en ce qu'elle présente une conductivité inférieure ou égale à 50 $\mu$S/cm lorsqu'elle est sous la forme d'une solution d'eau distillée à une concentration de 10 %.

De préférence, au moins 50 % des groupements méthyles de ladite méthyl-cyclodextrine sont situés au niveau de l'hydroxyle porté par le carbone C2 de l'unité glucopyranose.

De préférence, la méthyl-cyclodextrine est une méthyl-$\beta$-cyclodextrine.

De préférence, la méthyl-cyclodextrine est sous forme pulvérulente.

Facultativement, elle est sous forme amorphe.

Dans un mode de réalisation préféré, elle est sous la forme d'un produit atomisé.

La présente invention concerne également un procédé de préparation d'une méthyl-cyclodextrine présentant un MS compris entre 0,05 et 1,50, particulièrement utile pour la préparation de méthyl-cyclodextrines selon l'invention, comprenant l'étape de diminution des espèces ioniques de la méthyl-cyclodextrine, de manière à ce que la conductivité de ladite méthyl-cyclodextrine, lorsqu'elle est sous la forme d'une solution d'eau distillée à une concentration de 10 %, soit réduite à une valeur inférieure ou égale à 50 µS/cm.

De préférence, ce procédé comprend les étapes :

(a) d'éthérification d'une cyclodextrine avec un réactif de méthylation, ladite éthérification étant réalisée en milieu basique, préférentiellement aqueux, à une température comprise entre 100 et 200 °C, et à une passion comprise entre 1 et 10 bars ;

(b) de diminution des espèces ioniques de la méthyl-cyclodextrine obtenue à l'étape (a), de manière à ce que la conductivité de ladite méthyl-cyclodextrine, lorsqu'elle est sous la forme d'une solution d'eau distillée à une concentration de 10 %, soit réduite à une valeur inférieure ou égale à 50 µS/cm ;

(c) de séchage de la méthyl-cyclodextrine obtenue à l'étape (b) ;

(d) de récupération de la méthyl-cyclodextrine obtenue à l'étape (c).

De préférence, l'étape de diminution des espèces ioniques est réalisée en soumettant la méthyl-cyclodextrine en solution, en particulier en solution aqueuse, à :

- une opération (b.1) de nano-filtration de la solution de méthyl-cyclodextrine, ladite solution présentant une teneur en matières sèches en poids inférieure ou égale à 20% ;
- une opération (b.2) de déminéralisation sur colonne échangeuse d'ions ;
- une opération (b.3) de décoloration au charbon actif.

La présente invention concerne également une composition comprenant une méthyl-cyclodextrine selon l'invention, ou comprenant une méthyl-cyclodextrine obtenue selon le procédé de préparation d'une méthyl-cyclodextrine de l'invention. De préférence, la composition comprend en outre un composé lipophile ou porteur d'au moins un groupement lipophile.

La présente invention concerne en outre une composition de l'invention pour son utilisation comme médicament, de préférence pour son utilisation dans le traitement et/ou la prévention du diabète de type 2 et/ou de ses complications, et/ou de maladies susceptibles d'être traitées et/ou prévenues par une augmentation du taux de cholestérol-HDL et/ou par une réduction ou prévention des plaques d'athérome, et/ou de maladies du système nerveux central.

La présente invention concerne également l'utilisation d'une méthyl-cyclodextrine selon l'invention, ou d'une méthyl-cyclodextrine obtenue selon le procédé de préparation d'une méthyl-cyclodextrine de l'invention, pour la solubilisation de composés lipophiles, ou porteurs d'au moins un groupement lipophile, et/ou pour améliorer leur stabilité chimique, et/ou pour améliorer leur délivrance au niveau et à travers des membranes biologiques, et/ou pour augmenter leur stabilité physique, et/ou pour les convertir d'une forme liquide à une forme pulvérulente, et/ou pour prévenir les interactions avec d'autres composés, et/ou pour réduire l'irritation locale après une administration topique ou orale de ces composés lipophiles ou porteurs de groupements lipophiles, et/ou pour prévenir leur absorption au niveau des certains tissus comme la peau, et/ou pour obtenir une libération prolongé de ces composés, et/ou pour masque leur goût, en particulier leur amertume, et/ou pour modifier leur biodisponibilité.

**Description détaillée de l'invention**

Les méthyl-cyclodextrines de l'invention ont un bon pouvoir de solubilisation des agents lipophiles, ou porteurs de groupements lipophiles, tout en conservant un faible MS, lequel est garant de son activité pharmacologique sur le métabolisme lipidique.

Ce pouvoir de solubilisation est supérieur à celui de la MβCD employée dans la demande de brevet WO 2015/087016 A1. Il est également supérieur à celui des MβCD objets des brevets US 7,259,153 B2, US 6,602,860 B1 et US 5,935,941 A (voir l'Exemple ci-après).

Les méthyl-cyclodextrines de l'invention peuvent ainsi être avantageusement utilisées pour la solubilisation de principes actifs lipophiles ou porteurs de groupement lipophiles, dans des compositions pharmaceutiques. Dans ces compositions, la méthyl-cyclodextrine de l'invention peut avantageusement remplir la fonction d'agent d'encapsulation vis-à-vis de cet autre composé, et en outre, la fonction de principe actif.

Ainsi, la présente invention est relative à une composition pharmaceutique comprenant une méthyl-cyclodextrine selon la présente invention en tant que principe actif et/ou en tant qu'excipient. Cette composition pharmaceutique peut comprendre en outre un autre principe actif, de préférence lipophile ou porteur de groupements lipophiles, notamment des agents hypoglycémiants.

Parmi ces principes actifs lipophiles, il peut être fait mention des hypoglycémiants utilisés dans le diabète de type 2, qui pour la plupart sont très peu solubles dans l'eau.

L'utilisation des propriétés pharmacologiques de la méthyl-cyclodextrine serait également très avantageuse dans le cadre du traitement de cette pathologie puisque la majorité des patients atteints de diabète de type 2 présente, en plus d'une hyperglycémie caractéristique, des dyslipidémies mixtes. Ces dyslipidémies mixtes sont caractérisées par une diminution du taux de cholestérol-HDL, et une augmentation de la triglycéridémie, qui sont justement régulées par les méthyl-cyclodextrines de faible MS.

Un autre avantage de la méthyl-cyclodextrine de l'invention est que la forme amorphe ou cristalline de la méthyl-cyclodextrine de l'invention n'a aucune incidence sur son efficacité, ce qui laisse une grande liberté dans la texturation du produit et élargit le champ des formes galéniques possibles. Elle peut par exemple se présenter entièrement sous forme amorphe, par exemple sous la forme d'un produit atomisé.

La méthyl-cyclodextrine de l'invention est premièrement caractérisée par son degré de substitution molaire (MS), lequel est compris entre 0,05 et 1,50.

On rappelle ici que le « degré de substitution molaire (MS)» correspond au nombre de groupements hydroxyles substitués par un groupement méthyle, par unité glucopyranose. À noter que le degré de substitution molaire (MS) est différent du degré de substitution moléculaire (DS) qui correspond au nombre de groupements hydroxyles substitués par un groupement méthyle, par molécule de cyclodextrine et qui tient donc compte du nombre d'unités glucopyranoses constituant la méthyl-cyclodextrine.

Le MS peut être classiquement déterminé par l'homme du métier par Résonnance Magnétique Nucléaire du proton (RMN), ou par spectrométrie de masse (spectrométrie de masse par ionisation par électronébulisation (ESI-MS) ou spectrométrie de masse par désorption/ionisation par laser assisté par matrice (MALDI-MS)). Bien que ces techniques soient bien connues de l'homme du métier, ce dernier pourra par exemple se reporter aux méthodes décrites dans la thèse de référence de JACQUET Romain. « Cyclodextrines hydrophiles : caractérisation et étude de leurs propriétés énantiosélective et complexante. Utilisation de la chromatographie en phase liquide et de la spectrométrie de masse ». Thèse de Chimie et physicochimie des composés d'intérêt biologique. Université d'Orléans, 2006. notamment disponible sur : http://tel.archives-ouvertes.fr/docs/00/18/55/42/PDF/jacquet.pdf (consulté le 27.11.2013). », en particulier Chapitre 2, Partie B (pages 59 à 83).

De préférence, le MS est déterminé par RMN. On peut en particulier procéder selon la méthode décrite ci-après dans l'Exemple, au point B. 1.

Préférentiellement, le MS de la méthyl-cyclodextrine selon l'invention est compris entre 0,10 et 1,40, préférentiellement entre 0,10 et 1,30, préférentiellement entre 0,20 et 1,20, préférentiellement entre 0,30 et 1,10, préférentiellement entre 0,30 et 1,00, préférentiellement entre 0,50 et 0,90, préférentiellement entre 0,60 et 0,80, par exemple entre 0,60 et 0,70.

Il est à noter que la méthyl-cyclodextrine de l'invention, bien que pouvant correspondre à un produit pur, correspond généralement à un mélange de molécules de méthyl-cyclodextrine de structures différentes. Il en résulte que le MS mesuré est dans ce cas une moyenne des substitutions qui s'opèrent sur l'ensemble des unités glucopyranoses de l'ensemble du mélange de molécules de méthyl-cyclodextrine.

Ce mélange peut notamment contenir des molécules de cyclodextrine native résiduelles, c'est-à-dire non méthylées, mais qui se trouvent généralement et avantageusement en quantités négligeables au sein de la méthyl-cyclodextrine de l'invention. Préférentiellement, les cyclodextrines natives représentent moins de 1,0 % de la méthyl-cyclodextrine, préférentiellement moins de 0,5%, préférentiellement encore moins de 0,1% ; ces pourcentages étant exprimés en poids sec.

La méthyl-cyclodextrine selon l'invention est également caractérisée par sa conductivité, laquelle est inférieure ou égale à 50 µS/cm.

Cette conductivité est en particulier mesurée sur la base d'une solution d'eau distillée dans laquelle la méthyl-cyclodextrine est à une concentration de 10 %. Elle est en particulier mesurée à une température de 25°C. Elle peut aisément être déterminée par l'homme du métier, par exemple selon la méthode préconisée par la pharmacopée européenne de référence « 2.2.38. Conductivity, 01/2008 : 20238 », utilisée dans les Exemples ci-après.

Préférentiellement, la conductivité de la méthyl-cyclodextrine selon l'invention est comprise entre 0 et 45 µS/cm, préférentiellement entre 0 et 40 µS/cm, préférentiellement entre 0 et 35 µS/cm, préférentiellement entre 0 et 30 µS/cm, préférentiellement entre 0 et 25 µS/cm, préférentiellement entre 0 et 20 µS/cm. Cette conductivité est généralement d'au moins 1 µS/cm. Elle est par exemple choisie dans la gamme allant de 1 à 15 µS/cm, voire de 1 à 10 µS/cm, voire de 5 à 10 µS/cm, voire de 6 à 10 µS/cm, voire de 7 à 10 µS/cm, voire de 8 à 10 µS/cm.

La méthyl-cyclodextrine selon l'invention peut être substituée sur l'hydroxyle porté par le carbone C2 des unités glucopyranoses, ou par les carbones C3 et/ou C6 des unités glucopyranoses, ou par une combinaison des carbones C2, C3 et/ou C6, de préférence C2 et C6 des unités glucopyranoses.

Cette répartition des groupements méthyles sur les hydroxyles de l'unité glucopyranose de la méthyl-cyclodextrine peut être classiquement déterminée par l'homme du métier par RMN.

Préférentiellement, au moins 50 % des groupements méthyles de la méthyl-cyclodextrine de l'invention sont situés au niveau de l'hydroxyle porté par le carbone C2 de l'unité glucopyranose, par exemple 50 à 80 %, préférentiellement 60 à 80 %, préférentiellement 65 à 80 %, préférentiellement 70 à 80 %, par exemple 75 %.

Parallèlement, les autres groupements méthyles sont généralement majoritairement situés au niveau de l'hydroxyle porté par le carbone C3 et/ou C6 de l'unité glucopyranose.

Avantageusement, la méthyl-cyclodextrine de l'invention comporte 7 unités $\alpha$-D-glucopyranose. Il s'agit donc d'une méthyl-$\beta$-cyclodextrine.

Dans ce cas la méthyl-$\beta$-cyclodextrine présente préférentiellement le profil de substitution suivant :

- 0 à 5 % de méthyl-$\beta$-cyclodextrines comprennent 2 groupements méthyles (DS de 2) ;
- 5 à 15 % de méthyl-$\beta$-cyclodextrines comprennent 3 groupements méthyles (DS de 3) ;
- 20 à 25 % de méthyl-$\beta$-cyclodextrines comprennent 4 groupements méthyles (DS de 4) ;
- 25 à 40 % de méthyl-$\beta$-cyclodextrines comprennent 5 groupements méthyles (DS de 5) ;
- 15 à 25 % de méthyl-$\beta$-cyclodextrines comprennent 6 groupements méthyles (DS de 6) ;
- 5 à 15 % de méthyl-$\beta$-cyclodextrines comprennent 7 groupements méthyles (DS de 7) ;
- 0 à 5 % de méthyl-$\beta$-cyclodextrines comprennent 8 groupements méthyles (DS de 8) ;

ces pourcentages étant des pourcentages molaires, et leur somme totale étant généralement de l'ordre de 100%, bien que la composition puisse contenir éventuellement des traces de méthyl-cyclodextrines de DS différent, ainsi que des traces de cyclodextrine native, c'est-à-dire non méthylée.

Le profil de substitution peut être classiquement déterminé par l'homme du métier, par exemple par ESI-SM ou MALDI-TOF-SM. Bien que ces techniques soient bien connues de l'homme du métier, ce dernier pourra par exemple se reporter aux méthodes décrites dans la thèse de Romain JACQUET précitée, au chapitre 2, partie B, points II.3 et II.2 (page 67 à 82) et à l'Annexe II.

Généralement et avantageusement, la méthyl-cyclodextrine selon l'invention présente un taux de sucres réducteurs inférieur à 1,0 % en poids sec, préférentiellement inférieur à 0,5 %.

Généralement et avantageusement, la méthyl-cyclodextrine de l'invention comprend moins de 100 ppm en poids sec de phosphates, préférentiellement moins de 50 ppm, préférentiellement moins de 10 ppm, préférentiellement encore moins de 5 ppm.

Généralement et avantageusement, la méthyl-cyclodextrine de l'invention comprend moins de 20 ppm en poids sec d'agent de méthylation, notamment de diméthylsulfate, préférentiellement moins de 10 ppm, préférentiellement moins de 5 ppm, préférentiellement encore moins de 2 ppm.

Généralement et avantageusement, la méthyl-cyclodextrine de l'invention comprend moins de 1,0 % de sels d'halogénure de métal alcalin, préférentiellement moins de 0,5 %, préférentiellement moins de 0,2 %, préférentiellement encore moins de 0,1%; ce pourcentage étant exprimé en poids sec de sels d'halogénure de métal alcalin par rapport au poids sec total de ladite méthyl-cyclodextrine.

De préférence, la méthyl-cyclodextrine de l'invention comprend :

- moins de 100 ppm en poids sec de phosphates, préférentiellement moins de 50 ppm, préférentiellement moins de 10 ppm, préférentiellement encore moins de 5 ppm ;
- moins de 20 ppm en poids sec d'agent de méthylation, notamment de diméthylsulfate, préférentiellement moins de 10 ppm, préférentiellement moins de 5 ppm, préférentiellement encore moins de 2 ppm ; et
- moins de 1,0 % de sels d'halogénure de métal alcalin, préférentiellement moins de 0,5 %, préférentiellement moins de 0,2 %, préférentiellement encore moins de 0,1 % ; ce pourcentage étant exprimé en poids sec de sels d'halogénure de métal alcalin par rapport au poids sec total de ladite méthyl-cyclodextrine.

Généralement et avantageusement, la méthyl-cyclodextrine de l'invention a une absorbance à une longueur d'onde de 245 nm à 270 nm, inférieure à 0,5 A.U, préférentiellement inférieure à 0,3 A.U, préférentiellement encore inférieure à 0,2 A.U ; ladite absorbance étant déterminée par spectrophotométrie UV/vis, pour une solution aqueuse comprenant 100 mg de ladite méthyl-cyclodextrine par mL de solution, dans une cellule ayant une longueur de trajet d'1 cm.

Cette faible absorbance traduit avantageusement une réactivité minime de la méthyl-cyclodextrine vis-à-vis d'autres composés.

En effet les agents de dégradations des principes actifs pharmaceutiques présentent la particularité de présenter un maximum d'absorption à une longueur d'onde de 245 nm à 270 nm. Ce sont donc ces agents de dégradation que l'on mesure par spectrophotométrie à ces longueurs d'onde. Par « agent de dégradation des principes actifs pharmaceutiques » il faut en particulier entendre des composés, fragments, ou apparentés qui dégradent un principe actif pharmaceutique dans une solution aqueuse. Ces agents de dégradation comprennent typiquement des composés de poids moléculaire inférieur à 1 000 Da tels ceux générés par la préparation des méthyl-cyclodextrines, par exemple des fractions glycosidiques, des molécules de cyclodextrines décyclisées, des sucres réducteurs, des produits de dégradation du glucose tel du 3,4-dideoxyglucosone-3-ène, des composés porteurs de carbonyles tels que le 2-furaldéhyde, ou le 5-hydroxyméthyl-2-furaldéhyde.

Généralement et avantageusement, la méthyl-cyclodextrine de l'invention a une absorbance à une longueur d'onde de 320 nm à 350 nm, inférieure à 0,5 A.U, préférentiellement inférieure à 0,2 A.U, préférentiellement encore inférieure à 0,1 A.U ; ladite absorbance étant déterminée par spectrophotométrie UV/vis, pour une solution aqueuse comprenant 100 mg de ladite méthyl-cyclodextrine par mL de solution, dans une cellule ayant une longueur de trajet d'1 cm.

Cette absorbance, mesurée de 320 nm à 350 nm, permet en particulier de détecter les agents de coloration, lesquels sont susceptible d'interagir avec les actifs éventuellement présents.

La méthyl-cyclodextrine de l'invention peut être greffée en outre par d'autres groupements chimiques que des groupements méthyles, en faible proportion, et tant que cela ne contrevient pas aux propriétés recherchées dans la présente invention.

Préférentiellement, la méthyl-cyclodextrine de l'invention ne comprend pas d'autres groupements que des groupements méthyles. En particulier, elle est préférentiellement exempte de groupements hydroxyalkyles, en particulier de groupement hydroxyéthyles.

Dans un mode de réalisation particulier et avantageux, la méthyl-cyclodextrine de l'invention se présente sous forme pulvérulente. Elle peut dans ce cas se présenter sous forme amorphe, sous forme cristalline, ou sous la forme d'un mélange de ces deux formes. Elle est préférentiellement soit sous forme amorphe soit sous forme cristalline, c'est-à-dire que préférentiellement, elle n'est pas sous la forme d'un mélange de ces deux formes. Tout préférentiellement, la méthyl-cyclodextrine de l'invention est sous forme amorphe.

Avantageusement, la méthyl-cyclodextrine de l'invention se présente sous la forme d'un produit atomisé, c'est-à-dire sous la forme d'une poudre obtenue par séchage par atomisation d'une solution de cette méthyl-cyclodextrine, par exemple réalisé dans une tour d'atomisation simple-effet.

Avantageusement, la méthyl-cyclodextrine pulvérulente de l'invention présente un taux d'humidité inférieur à 10 %, par exemple inférieur à 5 %, par exemple compris entre 1 et 5 % ; ce pourcentage correspondant au poids d'eau par rapport au poids total de ladite méthyl-cyclodextrine pulvérulente.

L'invention concerne un procédé de préparation d'une méthyl-cyclodextrine présentant un MS compris entre 0,05 et 1,50, particulièrement utile pour la préparation de méthyl-cyclodextrines selon l'invention, comprenant l'étape de diminution des espèces ioniques de la méthyl-cyclodextrine, de manière à ce que la conductivité de ladite méthyl-cyclodextrine, lorsqu'elle est sous la forme d'une solution d'eau distillée à une concentration de 10 %, soit réduite à une valeur inférieure ou égale à 50 $\mu$S/cm.

Plus particulièrement, l'invention concerne aussi un procédé de préparation d'une méthyl-cyclodextrine présentant un MS compris entre 0,05 et 1,50, particulièrement utile pour la préparation de méthyl-cyclodextrines selon l'invention, comprenant les étapes :

(a) d'éthérification d'une cyclodextrine avec un réactif de méthylation, ladite éthérification étant réalisée en milieu basique, préférentiellement aqueux, à une température comprise entre 100 et 200 °C, et à une pression comprise entre 1 et 10 bars ;
(b) de diminution des espèces ioniques de la méthyl-cyclodextrine obtenue à l'étape (a), de manière à ce que la conductivité de ladite méthyl-cyclodextrine, lorsqu'elle est sous la forme d'une solution d'eau distillée à une concentration de 10 %, soit réduite à une valeur inférieure ou égale à 50 $\mu$S/cm ;
(c) de séchage de la méthyl-cyclodextrine obtenue à l'étape (b) ;
(d) de récupération de la méthyl-cyclodextrine obtenue à l'étape (c).

Préférentiellement, le réactif de méthylation auquel se réfère l'étape (a) est le diméthylsulfate.

Préférentiellement, l'étape (a) d'éthérification est réalisée à une température comprise entre 90 et 190°C, préférentiellement entre 100 et 180°C, préférentiellement entre 110 et 170°C, préférentiellement entre 120 et 160°C, préfréentiellement entre 130 et 150°C, par exemple égale à 140°C.

Préférentiellement, l'étape (a) d'éthérification est réalisée à une pression comprise entre 2 et 9 bars, préférentiellement entre 2 et 8 bars, préférentiellement entre 2 et 7 bars, préférentiellement entre 2 et 6 bars, préférentiellement entre 3 et 5 bars, par exemple égale à 4 bars.

Le milieu réactionnel de l'étape (a) peut avantageusement être rendu basique par addition d'hydroxyde de calcium. La réaction peut ensuite par exemple être neutralisée à l'acide sulfurique.

L'étape (b) peut en particulier être réalisée en soumettant la méthyl-cyclodextrine en solution, en particulier en solution aqueuse, à :

- une opération (b.1) de nano-filtration de la solution de méthyl-cyclodextrine, ladite solution présentant une teneur en matières sèches en poids inférieure ou égale à 20% ;

- une opération (b.2) de déminéralisation sur colonne échangeuse d'ions ;
- une opération (b.3) de décoloration au charbon actif ;

lesdites étapes (b.1), (b.2) et (b.3) étant préférentiellement réalisées dans cet ordre ou dans l'ordre suivant : (b.1), (b.3) et (b.2).

L'étape (b) peut être précédée d'une étape de filtration ou d'essorage et de lavage, en particulier afin d'éliminer le sulfate de calcium susceptible de se former (en fonction du réactif de méthylation choisi, ainsi que de l'acide utilisé pour neutraliser la réaction).

Préférentiellement, l'étape (c) de séchage est une étape de séchage par atomisation.

Cette atomisation peut être une atomisation simple-effet ou multiple-effet. Dans le cas d'une atomisation multiple-effet, l'atomiseur est couplé avec un lit fluidisé, éventuellement intégré à la tour d'atomisation, qui permet d'agglomérer les particules formées par atomisation. Ce dernier procédé est particulièrement intéressant si l'on souhaite d'obtenir des poudres de diamètre moyen supérieur, et en fonction de l'écoulement souhaité pour la poudre résultante.

L'invention concerne également une composition comprenant une méthyl-cyclodextrine selon l'invention, ou comprenant une méthyl-cyclodextrine obtenue selon le procédé de préparation d'une méthyl-cyclodextrine de l'invention.

La composition selon l'invention comprend préférentiellement en outre, au moins un composé lipophile ou porteur d'au moins un groupement lipophile.

Il peut par exemple s'agir de composés hydrophobe, c'est-à-dire classiquement de composés peu solubles, très peu solubles, voire pratiquement insolubles dans l'eau, à température ambiante (15-25°C). Par «composé peu soluble dans l'eau », on entend classiquement qu'un volume d'eau de 100 à 1 000 mL est nécessaire pour dissoudre 1 gramme dudit composé. Pour un « composé très peu soluble dans l'eau », ce volume d'eau est de plus de 1 000 mL et va jusqu'à 10 000 mL. Pour un « composé pratiquement insoluble dans l'eau », ce volume d'eau est de plus de 10 000 mL. À ce sujet, voir en particulier la définition donnée dans la Pharmacopée Européenne de référence « 1.4. Monographs, 07/2014 : 10000 ».

Préférentiellement, le composé lipophile ou porteur d'au moins un groupement lipophile auquel se réfère l'invention est un principe actif pharmaceutique, préférentiellement choisi parmi les hypoglycémiants et leurs mélanges. Les hypoglycémiants comprennent essentiellement la metformine, les sulfonylurées tels que la glibenclamide, la gliclazide, la glimépiride, la glipizide, ou la gliquidone, les glitazones, les gliptines, les agonistes des GLP-1 (Glucagon-Like Petide-1), tels que l'exénatide et le liraglutide, l'acarbose, le natéglinide et le répaglinide. Préférentiellement, cet hypoglycémiant est choisi parmi les sulfonylurées et il s'agit tout préférentiellement de la glipizide. Le principe actif pharmaceutique en question peut également être choisi parmi les statines, les agents anti-hypertenseurs, les antagonistes du récepteur à l'angiotensine II, aussi connus sous le nom de « sartans », les beta-bloquants, les antiagrégants plaquettaires ou anticoagulants.

Préférentiellement, les compositions de l'invention sont des compositions pharmaceutiques ou des compositions pour leur utilisation comme médicament, par exemple pour une utilisation dans le traitement et/ou la prévention du diabète de type 2 et/ou de ses complications. Les compositions de l'invention étant également capable d'agir sur le métabolisme lipidique, elles peuvent également être utilisées pour le traitement et/ou la prévention de maladies susceptibles d'être traitées et/ou prévenues par une augmentation du taux de cholestérol-HDL et/ou par une réduction ou prévention des plaques d'athérome, en particulier de l'athérosclérose ou des complications liées à un athérome, et/ou des maladies du système nerveux central, en particulier la maladie d'Alzheimer, de Parkinson ou de Niemann Pick de type C.

Les maladies susceptibles d'être traitées et/ou prévenues par une augmentation du taux de cholestérol-HDL sont typiquement les maladies liées à une surcharge, et/ou à un stockage, et/ou à l'accumulation de cholestérol dans les tissus, ainsi que leurs conséquences. Cela inclut par exemple les maladies cardiovasculaires, les maladies vasculaires, les maladies artérielles périphériques occlusives telles que l'athérosclérose ou les complications liées à un athérome, les maladies du système nerveux central comme la maladie d'Alzheimer, la maladie de Parkinson, et les maladies lysosomale affectant le système nerveux central comme par exemple la maladie de Niemann-Pick, telle que la maladie de Niemann-Pick type A, la maladie de Niemann-Pick type B, ou la maladie de Niemann-Pick type C. Les complications liées à un athérome qui sont traitées et/ou prévenues par l'utilisation d'une composition pharmaceutique selon l'invention sont de manière non limitative l'ischémie, par exemple l'ischémie du myocarde, les maladies coronariennes, l'angine de poitrine, le syndrome coronarien aigu, l'infarctus du myocarde, l'infarctus mésentérique, l'accident vasculaire-cérébral, l'anévrisme ou l'artériopathie des membres inférieurs.

La présente invention concerne également l'utilisation d'une composition pharmaceutique selon la présente invention pour la fabrication d'un médicament, notamment destiné au traitement et/ou à la prévention des conditions et pathologies précitées. Elle est également relative à une méthode de traitement et/ou de prévention des conditions et pathologies précitées chez un sujet comprenant l'administration d'une quantité thérapeutiquement efficace d'une composition pharmaceutique selon la présente invention.

Les compositions de l'invention peuvent être sous toute forme galénique que l'homme du métier juge adaptée, en fonction de l'utilisation visée. Elles peuvent par exemple être sous forme liquide, solide, ou semi-solide. Il peut par exemple s'agir de solutions, de suspensions, de dispersions, d'émulsions, de « pellet », de granules, de films, de poudres, de gels, de crèmes, d'onguents, de pâtes, de sticks, de comprimés, de gélules dures, de capsules molles, de dispositifs osmotiques, de patchs.

Les compositions de l'invention peuvent par ailleurs comprendre tout autre composé additionnel et habituel, tant que cela ne contrevient pas aux propriétés recherchées dans la présente invention. Ces composés sont typiquement choisis en fonction de la forme galénique sélectionnée pour la composition.

Les compositions de l'invention sont susceptibles d'être administrées par voie orale, par voie parentérale, ou par voie cutanée ou mucosale. La voie parentérale comprend par exemple l'administration sous-cutanée, intraveineuse, intramusculaire ou intrapéritonéale, bien que cette dernière soit plutôt réservée à l'animal. La voie mucosale comprend par exemple l'administration par voie nasale, par voie pulmonaire, par la muqueuse rectale. La voie cutanée comprend par exemple la voie dermique, notamment via un dispositif transdermique, typiquement un patch. Pour le traitement et/ou la prévention des maladies du système nerveux central, la voie intrathécale ou la voie rachidienne sont également susceptibles d'être employées.

Enfin, l'invention concerne l'utilisation d'une méthyl-cyclodextrine selon l'invention, ou d'une méthyl-cyclodextrine obtenue selon le procédé de préparation d'une méthyl-cyclodextrine de l'invention, pour la solubilisation en milieu aqueux de composés lipophiles ou porteurs d'au moins un groupement lipophile, ledit composé étant préférentiellement tel que défini avant.

L'invention concerne également l'utilisation d'une méthyl-cyclodextrine selon l'invention, ou d'une méthyl-cyclodextrine obtenue selon le procédé de préparation d'une méthyl-cyclodextrine de l'invention, pour améliorer la stabilité chimique de composés lipophiles ou porteurs d'au moins un groupement lipophile, et/ou pour améliorer leur délivrance au niveau et à travers des membranes biologiques, et/ou pour augmenter leur stabilité physique, et/ou pour les convertir d'une forme liquide à une forme pulvérulente, et/ou pour prévenir les interactions avec d'autres composés, et/ou pour réduire l'irritation locale après une administration topique ou orale de ces composés lipophiles ou porteurs de groupements lipophiles, et/ou pour prévenir leur absorption au niveau des certains tissus comme la peau, et/ou pour obtenir une libération prolongé de ces composés, et/ou pour masque leur goût, en particulier leur amertume, et/ou pour modifier leur biodisponibilité.

Notons que dans la présente invention, il est entendu que l'expression « compris entre X et Y » couvre une plage de valeurs excluant les bornes citées.

Il est par ailleurs entendu que lorsque l'on se réfère à la concentration en pourcentage d'une substance en solution, cette concentration exprime le nombre de grammes de ladite substance pour 100 mL de ladite solution. Cette masse en grammes est bien une masse sèche, c'est-à-dire qu'elle exclue notamment la masse d'eau éventuellement présente dans la substance sous sa forme pulvérulente, avant solubilisation.

L'invention sera mieux comprise à l'aide des exemples qui suivent, lesquels se veulent illustratifs et non limitatifs.

**Figure**

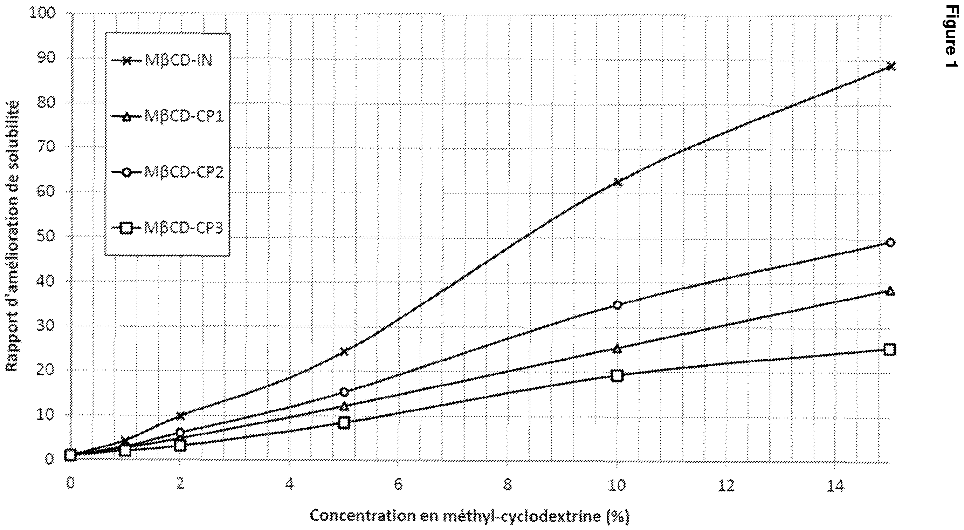

La figure 1 décrit le rapport d'amélioration de la solubilité en fonction de la concentration en méthyl-cyclodextrine pour plusieurs échantillons de méthyl-cyclodextrine (« MβCD-IN » « MβCD-CP1 », « MβCD-CP2 » et « MβCD-CP3 »).

**Exemple**

Dans les essais qui suivent, une méthyl-β-cyclodextrine selon l'invention (« MβCD-IN ») a été comparée à des méthyl-β-cyclodextrines utilisant des procédés de l'art antérieur (« MβCD-CP1 », « MβCD-CP2 » et « MβCD-CP3 »).

Les inventeurs ont fait en sorte que ces MβCD présentent toutes un MS de 0,7, de manière à pouvoir comparer efficacement ces MβCD sur la base du critère de conductivité.

**A. Méthyl-β-cyclodextrines (MBCD) utilisées**

**1. Préparation d'une MβCD selon l'invention (MβCD-IN)**

Une β-cyclodextrine native a été éthérifiée au diméthylsulfate, en milieux aqueux et en présence d'hydroxyde de calcium, dans les conditions suivantes de température et de pression : 140°C / 4 bars. Le milieu réactionnel a ensuite été neutralisé à l'acide sulfurique.

Le produit réactionnel a ensuite été essoré (essoreuse Dorr-Oliver BW630H) et lavé, afin d'éliminer le sulfate de calcium formé pendant la réaction. Le lavage a en particulier été réalisé par buse de pulvérisation avec eau déminéralisée chauffée à 70°C. 3,5 tonnes de produit réactionnel à 23 % de teneur en matières sèches ont ainsi été obtenus.

Les espèces ioniques du produit réactionnel ainsi obtenu ont été séparées par les opérations suivantes :

- nanofiltration de la solution ajustée à 20 % de teneur en matières sèches ;
- déminéralisation sur colonne échangeuse d'ions ;
- décoloration au charbon actif.

L'étape de nanofiltration a en particulier été réalisée dans les conditions suivantes : utilisation de membranes AFC30 ; utilisation de deux tubes en parallèle de 1,7 m$^2$ (3,4 m$^2$ au total) ; pompe haute pression de 2,7 m$^2$/h ; contre pression régulée à 20 bars entrée module ; solution de MβCD ajustée à 20 % de teneur en matières sèches, amenée à une température de 55°C et à un pH de 5,5 ; débit de perméat de 70 à 85 L/h/m$^2$; pression de sortie de 18 bars. 6 filtrations ont ainsi été réalisées.

L'étape de déminéralisation a en particulier été réalisée dans les conditions suivantes :

- traitement à température inférieure à 40°C, 50 L/h, à 20 % de teneur en matières sèches :

  ◦ cationique (Amberlite 252) ; 25 litres ; 1,8 eq/L ;
  ◦ anionique (Amberlite IRA 910) ; 40 litres ; 1,1 eq/L ;
  ◦ lit mixte ; 30 litres ;

2 cuves de 700 L ont été déminéralisées avant régénération.

L'étape de décoloration au charbon actif (NORIT SX plus) a en particulier été réalisée dans les conditions suivantes : solution de MβCD à 20% de teneur en matières sèches traitée avec 1 % de noir ; agitation 1 heure ; pH 5 - 5,5 ; température ambiante ; filtration sur manchette de 11 μm, puis de 8 μm et enfin de 0,22 μm.

La MβCD ainsi obtenue a ensuite été concentrée de manière à présenter une teneur en matières sèches en poids de 30 %, à l'aide d'un évaporateur (NIRO), en particulier dans les conditions suivantes: débit d'alimentation de 100 L/h ; température du produit de 62°C ; Pression de 200 mbars.

La solution ainsi obtenue a été séchée par atomisation simple-effet (atomiseur NIRO). Les conditions étaient en particulier les suivantes : solution à atomiser ajustée à une température de 70°C et à un pH de 6,5 - 7 ; filtrafon de la solution (0,22 μm) ; température de l'air d'entrée de 250°C ; température de l'air de sortie régulée à 115°C ; débit d'air de 120 Nm$^3$/h ; dépression ajustée à 35 mm d'eau ; débit du produit atomisé de 6,8 à 7 kg/h.

La MβCD pulvérulente ainsi obtenue (MβCD-IN) présentait un taux d'humidité de 3,5%, un taux de sucres réducteurs de 0,3 % et une teneur en β-cyclodextrine native résiduelle inférieure à 0,1 %.

## 2. MβCD comparatives (MβCD-CP1, -CP2 et -CP3)

La méthyl-cyclodextrine comparative « MβCD-CP1 » correspond à celle employée dans le brevet US 7,259,153 B2 et désignée sous le terme « Cryst. Methylated β-CD » dans ce brevet. Bien qu'aucun procédé de préparation de cette MβCD ne soit décrit dans ce brevet, et que ce produit ne soit pas disponible dans le commerce, les inventeurs sont partis du principe qu'elle était préparée selon le brevet US 5,935,941 A. En effet, ce brevet est cité comme base prometteuse pour la préparation de la MβCD du brevet US 7,259,153 B2. Les inventeurs se sont en particulier basés sur l'Exemple 15 du brevet US 5,935,941 A, seul exemple illustrant la préparation de cylodextrines méthylées. Le produit réactionnel a en particulier été traité selon les méthodes décrites à l'Exemple 1 de ce document.

La méthyl-cyclodextrine comparative « MβCD-CP2 » a été préparée selon le brevet US 6,602,860 B1. Le produit réactionnel a en particulier été traité selon les méthodes décrites à l'Exemple 1 de ce document.

La méthyl-cyclodextrine comparative « MβCD-CP3 » correspond au produit commercialisé sous la dénomination KLEPTOSE® Crysmeb, utilisé dans la demande de brevet WO 2015/087016 A1. Ce produit est en particulier obtenu selon le brevet US 9,935,941 A, à l'exception du fait qu'il est séché par atomisation.

## B. Caractérisation

Les caractéristiques des méthyl-cyclodextrines MβCD-IN, MβCD-CP1, MβCD-CP2 et MβCD-CP3 ont été déterminées selon les méthodes suivantes.

1. Détermination du degré de substitution molaire (MS) et du profil de substitution. Le MS a été déterminé par RMN du proton (sur un appareil DPX 250 MHz Advance (Bruker, Rheinstetten, Allemagne)). Les mesures ont été conduites à 25°C. La calibration a été effectuée avec le signal D2O. Les échantillons de MβCD, et de cyclodextrine native, c'est-à-dire non méthylée, ont été préparés à une concentration de 5 mg dans 0,75 mL de D$_2$O. Les solutions ont été évaporées à sec sous courant d'azote puis reconstituées dans 0,75 mL de D$_2$O. Cette opération a été répétée deux fois afin d'assurer un échange total des protons des fonctions hydroxyles. Le MS a été calculé à partir de la différence d'intégration entre le spectre de la cyclodextrine native et celui de la méthyl-cyclodextrine conforme à l'invention. Le spectre RMN a également permis le calcul du profil de substitution.

2. Détermination de la conductivité. La conductivité a été déterminée à 25°C selon la méthode préconisée par la pharmacopée européenne de référence « 2.2.38. Conductivity, 01/2008 : 20238 », sur la base d'une solution de 100 mL, à 10 % de MβCD. En particulier, 10 grammes secs de MβCD ont été placés dans une fiole jaugée de 100 mL. De l'eau distillée présentant une résistivité supérieure à 500 000 ohms.cm a été ajoutée (q.s.p. 100 mL).

La conductivité de cette solution a en particulier été déterminée à l'aide d'un conductivimètre électronique (KNICK 703) équipé de sa cellule de mesure et vérifié selon le mode opératoire décrit dans l'instruction s'y rapportant.

Les résultats obtenus sont présentés dans le Tableau 1.

Tableau 1

| MβCD | MβCD-IN | MβCD-CP1 | MβCD-CP2 | MβCD-CP3 |
|---|---|---|---|---|
| Degré de substitution molaire (MS) | 0,67 | 0,67 | 0,68 | 0,67 |
| Profil de substitution | 75 % des substitutions portées par des carbones C2 | ND | ND | ND |
| Conductivité | 9 μS/cm | 89 μS/cm | 62 μS/cm | 102 μS/cm |
| ND : non déterminé | | | | |

## C. Test de solubilisation de la glipizide

La méthyl-cyclodextrine MβCD-IN selon l'invention et les méthyl-cyclodextrines comparatives MβCD-CP1, MβCD-CP2 et MβCD-CP3 ont été évaluées pour leur capacité à solubiliser la glipizide dans de l'eau distillée, selon la méthode suivante : 6 solutions à 0, 1, 2, 10 et 15 % de MβCD ont été préparées, pour chaque MβCD. De l'eau distillée a été utilisée comme solution témoin. Ces solutions ont été placées sous agitation à température ambiante. De la glipizide a été ajoutée milligramme par milligramme, jusqu'à ce qu'elle ne se solubilise plus. La quantité limite de glipizide se solubilisant a été notée pour chaque solution, ce qui a permis de calculer le nombre de mg de glipizide solubilisée par mL de solution.

Le rapport d'amélioration de la solubilité donne le facteur par lequel la solubilité est augmentée en présence d'une concentration définie en méthyl-cyclodextrine. Il est calculé comme suit :

$$Rapport\ de\ solubilité = \frac{S_{MCD}}{S_{H2O}}$$

avec : « $S_{MCD}$ » exprimée mg de glipizide solubilisée par mL de solution de méthyl-cyclodextrine ; « $S_{H2O}$ » exprimée mg de glipizide solubilisée par mL d'eau.

Les résultats sont présentés Figure 1.

Il est constaté que le rapport d'amélioration de solubilité est bien plus élevé pour ce qui concerne la méthyl-cyclodextrine MβCD-IN selon l'invention, comparativement au méthyl-cyclodextrines MβCD-CP1, MβCD-CP2 et MβCD-3, non conformes à l'invention.

Il peut être déduit qu'à de faibles MS, une méthyl-cyclodextrine présentant une conductivité inférieure ou égale à 50 μS/cm, telle que la méthyl-cyclodextrine MβCD-IN selon l'invention, est un bien meilleur solubilisant des composés lipophiles ou des composés porteurs de groupements lipophiles.

## Revendications

1. Méthyl-cyclodextrine présentant un degré de substitution molaire (MS) compris entre 0,05 et 1,50, **caractérisée en ce qu'**elle présente une conductivité inférieure ou égale à 50 μS/cm lorsqu'elle est sous la forme d'une solution d'eau distillée à une concentration de 10 %.

2. Méthyl-cyclodextrine selon la revendication 1, **caractérisée en ce qu'**au moins 50 % des groupements méthyles de ladite méthyl-cyclodextrine sont situés au niveau de l'hydroxyle porté par le carbone C2 de l'unité glucopyranose.

**3.** Méthyl-cyclodextrine selon la revendication l'une ou l'autre des revendications 1 et 2, **caractérisée en ce qu'**il s'agit d'une méthyl-β-cyclodextrine.

**4.** Méthyl-cyclodextrine selon l'une quelconque des revendications 1 à 3, **caractérisée en ce qu'**elle est sous forme pulvérulente.

**5.** Méthyl-cyclodextrine pulvérulente selon la revendication 4, **caractérisée en ce qu'**elle est sous forme amorphe.

**6.** Méthyl-cyclodextrine selon l'une quelconque des revendications 1 à 5, **caractérisée en ce qu'**elle est sous la forme d'un produit atomisé.

**7.** Procédé de préparation d'une méthyl-cyclodextrine présentant un MS compris entre 0,05 et 1,50, comprenant l'étape de diminution des espèces ioniques de la méthyl-cyclodextrine, de manière à ce que la conductivité de ladite méthyl-cyclodextrine, lorsqu'elle est sous la forme d'une solution d'eau distillée à une concentration de 10 %, soit réduite à une valeur inférieure ou égale à 50 µS/cm.

**8.** Procédé de préparation d'une méthyl-cyclodextrine présentant un degré de substitution molaire (MS) compris entre 0,05 et 1,50, comprenant les étapes :

(a) d'éthérification d'une cyclodextrine avec un réactif de méthylation, ladite éthérification étant réalisée en milieu basique à une température comprise entre 100 et 200°C, et à une pression comprise entre 1 et 10 bars ;
(b) de diminution des espèces ioniques de la méthyl-cyclodextrine obtenue à l'étape (a), de manière à ce que la conductivité de ladite méthyl-cyclodextrine, lorsqu'elle est sous la forme d'une solution d'eau distillée à une concentration de 10 %, soit réduite à une valeur inférieure ou égale à 50 µS/cm ;
(c) de séchage de la méthyl-cyclodextrine obtenue à l'étape (b) ;
(d) de récupération de la méthyl-cyclodextrine obtenue à l'étape (c).

**9.** Procédé de préparation d'une méthyl-cyclodextrine selon l'une ou l'autre des revendications 7 et 8, **caractérisé en ce que** l'étape de diminution des espèces ioniques est réalisée en soumettant la méthyl-cyclodextrine en solution à :

- une opération (b.1) de nano-filtration de la solution de méthyl-cyclodextrine, ladite solution présentant une teneur en matières sèches en poids inférieure ou égale à 20% ;
- une opération (b.2) de déminéralisation sur colonne échangeuse d'ions ;
- une opération (b.3) de décoloration au charbon actif.

**10.** Composition comprenant une méthyl-cyclodextrine telle que définie dans l'une quelconque des revendications 1 à 6, ou comprenant une méthyl-cyclodextrine obtenue selon le procédé tel que défini dans l'une quelconque des revendications 7 à 9.

**11.** Composition selon la revendication 10 comprenant en outre un composé lipophile ou porteur d'au moins un groupement lipophile.

**12.** Composition selon l'une ou l'autre des revendications 10 et 11, pour son utilisation comme médicament.

**13.** Composition selon la revendication 12, pour son utilisation dans le traitement et/ou la prévention du diabète de type 2 et/ou de ses complications, et/ou de maladies susceptibles d'être traitées et/ou prévenues par une augmentation du taux de cholestérol-HDL et/ou par une réduction ou prévention des plaques d'athérome, et/ou de maladies du système nerveux central.

**14.** Utilisation d'une méthyl-cyclodextrine telle que définie dans l'une quelconque des revendications 1 à 6, ou d'une méthyl-cyclodextrine obtenue selon le procédé tel que défini dans l'une quelconque des revendications 7 à 9, pour la solubilisation de composés lipophiles, ou porteurs d'au moins un groupement lipophile, et/ou pour améliorer leur stabilité chimique, et/ou pour améliorer leur délivrance au niveau et à travers des membranes biologiques, et/ou pour augmenter leur stabilité physique, et/ou pour les convertir d'une forme liquide à une forme pulvérulente, et/ou pour prévenir les interactions avec d'autres composés, et/ou pour réduire l'irritation locale après une administration topique ou orale de ces composés lipophiles ou porteurs de groupements lipophiles, et/ou pour prévenir leur absorption au niveau des certains tissus comme la peau, et/ou pour obtenir une libération prolongé de ces composés, et/ou pour masque leur goût, et/ou pour modifier leur biodisponibilité.

**EP 3 362 487 B1**

**Patentansprüche**

1. Methylcyclodextrin mit einem molaren Substitutionsgrad (MS) zwischen 0,05 und 1,50, **dadurch gekennzeichnet, dass** es eine Leitfähigkeit von 50 $\mu$S/cm oder weniger aufweist, wenn es in Form einer Lösung von destilliertem Wasser mit einer Konzentration von 10 % vorliegt.

2. Methylcyclodextrin nach Anspruch 1, **dadurch gekennzeichnet, dass** sich mindestens 50 % der Methylgruppen des Methylcyclodextrins an der Hydroxylgruppe befinden, die vom C2-Kohlenstoff der Glucopyranose-Einheit getragen wird.

3. Methylcyclodextrin nach einem der Ansprüche 1 und 2, **dadurch gekennzeichnet, dass** es sich um ein Methyl-$\beta$-cyclodextrin handelt.

4. Methylcyclodextrin nach einem der Ansprüche 1 bis 3, **dadurch gekennzeichnet, dass** es in Pulverform vorliegt.

5. Pulverförmiges Methyl-Cyclodextrin nach Anspruch 4, **dadurch gekennzeichnet, dass** es in amorpher Form vorliegt.

6. Methylcyclodextrin nach einem der Ansprüche 1 bis 5, **dadurch gekennzeichnet, dass** es in Form eines zerstäubten Produkts vorliegt.

7. Verfahren zur Herstellung eines Methylcyclodextrins mit einem MS zwischen 0,05 und 1,50, umfassend den Schritt der Verringerung der ionischen Spezies des Methylcyclodextrins, so dass die Leitfähigkeit des Methylcyclodextrins, wenn es in Form einer Lösung von destilliertem Wasser mit einer Konzentration von 10 % vorliegt, auf einen Wert von 50 $\mu$S/cm oder weniger verringert wird.

8. Verfahren zur Herstellung eines Methylcyclodextrins mit einem molaren Substitutionsgrad (MS) zwischen 0,05 und 1,50, umfassend die Schritte:

   (a) Veretherung eines Cyclodextrins mit einem Methylierungsreagenz, wobei die Veretherung in einem basischen Milieu bei einer Temperatur zwischen 100 und 200°C und einem Druck zwischen 1 und 10 bar durchgeführt wird;
   (b) Verringerung der ionischen Spezies des in Schritt (a) erhaltenen Methylcyclodextrins, so dass die Leitfähigkeit des Methylcyclodextrins, wenn es in Form einer Lösung von destilliertem Wasser mit einer Konzentration von 10 % vorliegt, auf einen Wert von 50 $\mu$S/cm oder weniger verringert wird;
   (c) Trocknung des in Schritt (b) erhaltenen Methylcyclodextrins;
   (d) Gewinnung des in Schritt (c) erhaltenen Methylcyclodextrins.

9. Verfahren zur Herstellung eines Methylcyclodextrins nach einem der Ansprüche 7 und 8, **dadurch gekennzeichnet, dass** der Schritt der Verringerung der ionischen Spezies durchgeführt wird, indem das Methylcyclodextrin in Lösung:

   - einem Vorgang (b.1) der Nanofiltration der Methylcyclodextrin-Lösung, wobei die Lösung einen Trockenmassegehalt von 20 Gew.-% oder weniger aufweist;
   - einem Vorgang (b.2) der Entmineralisierung über eine Ionenaustauschsäule;
   - einem Vorgang (b.3) der Entfärbung mit Aktivkohle

   unterzogen wird.

10. Zusammensetzung, die ein Methylcyclodextrin, wie in einem der Ansprüche 1 bis 6 definiert, umfasst oder die ein Methylcyclodextrin umfasst, das nach dem Verfahren, wie in einem der Ansprüche 7 bis 9 definiert, erhalten wurde.

11. Zusammensetzung nach Anspruch 10, die zusätzlich eine lipophile Verbindung oder eine Verbindung, die mindestens eine lipophile Gruppe trägt, umfasst.

12. Zusammensetzung nach einem der Ansprüche 10 und 11, zur Verwendung als Arzneimittel.

13. Zusammensetzung nach Anspruch 12, zur Verwendung bei der Behandlung und/oder Vorbeugung von Typ-2-Diabetes und/oder seinen Komplikationen und/oder von Krankheiten, die durch eine Erhöhung des HDL-Cholesterinspiegels und/oder durch eine Verringerung oder Vorbeugung von atherosklerotischen Plaques behandelt und/oder verhindert werden können, und/oder von Krankheiten des zentralen Nervensystems.

14. Verwendung eines Methylcyclodextrins, wie in einem der Ansprüche 1 bis 6 definiert, oder eines Methylcyclodextrins, das nach dem Verfahren, wie in einem der Ansprüche 7 bis 9 definiert, erhalten wurde, zur Solubilisierung von Verbindungen, die lipophil sind oder mindestens eine lipophile Gruppe tragen, und/oder zur Verbesserung ihrer chemischen Stabilität, und/oder zur Verbesserung ihrer Abgabe an und durch biologische Membranen, und/oder zur Erhöhung ihrer physikalischen Stabilität, und/oder um sie von einer flüssigen in eine pulverförmige Form zu überführen, und/oder um ihre Wechselwirkungen mit anderen Verbindungen zu verhindern, und/oder um die lokale Reizung nach topischer oder oraler Verabreichung dieser lipophilen oder lipophile Gruppen tragenden Verbindungen zu verringern, und/oder um ihre Absorption in bestimmten Geweben wie der Haut zu verhindern, und/oder um eine verlängerte Freisetzung dieser Verbindungen zu erreichen, und/oder um ihren Geschmack zu maskieren, und/oder um ihre Bioverfügbarkeit zu verändern.

## Claims

1. Methyl cyclodextrin having a degree of molar substitution (MS) of between 0.05 and 1.50, **characterised in that** it has a conductivity less than or equal to 50 $\mu$S/cm when it is in the form of a distilled-water solution at a concentration of 10%.

2. Methyl cyclodextrin according to claim 1, **characterised in that** at least 50% of the methyl groups of said methyl cyclodextrin are located at the hydroxyl carried by the C2 carbon of the glucopyranose unit.

3. Methyl cyclodextrin according to either one of claims 1 and 2, **characterised in that** it is a methyl-$\beta$-cyclodextrin.

4. Methyl cyclodextrin according to any one of claims 1 to 3, **characterised in that** it is in powdery form.

5. Powdery methyl cyclodextrin according to claim 4, **characterised in that** it is in amorphous form.

6. Methyl cyclodextrin according to any one of claims 1 to 5, **characterised in that** it is in the form of an atomised product.

7. Method for preparing a methyl cyclodextrin having an MS of between 0.05 and 1.50, comprising the step of reducing the ionic species of the methyl cyclodextrin, so that the conductivity of said methyl cyclodextrin, when it is in the form of a distilled-water solution at a concentration of 10%, is reduced to a value below or equal to 50 $\mu$S/cm.

8. Method for preparing a methyl cyclodextrin having a degree of molar substitution (MS) of between 0.05 and 1.50, comprising the steps of:

   (a) etherification of a cyclodextrin with a methylation reagent, said etherification being implemented in a basic medium at a temperature of between 100 and 200°C, and at a pressure of between 1 and 10 bar;
   (b) reducing the ionic species of the methyl cyclodextrin obtained at step (a), so that the conductivity of said methyl cyclodextrin, when it is in the form of a distilled-water solution at a concentration of 10%, is reduced to a value below or equal to 50 $\mu$S/cm;
   (c) drying the methyl cyclodextrin obtained at step (b);
   (d) recovering the methyl cyclodextrin obtained at step (c).

9. Method for preparing a methyl cyclodextrin according to one or other of claims 7 and 8, **characterised in that** the step of reducing the ionic species is implemented by subjecting the methyl cyclodextrin in solution to:

   - an operation (b.1) of nanofiltration of the methyl cyclodextrin solution, said solution having a weight proportion of dry matter of less than or equal to 20%;
   - an operation (b.2) of demineralisation on ion exchange column;
   - an operation (b.3) of decolouring by active carbon.

10. Composition comprising a methyl cyclodextrin as defined in any one of claims 1 to 6, or comprising a methyl cyclodextrin obtained according to the method as defined in any one of claims 7 to 9.

**11.** Composition according to claim 10 further comprising a lipophilic compound or one carrying at least one lipophilic group.

**12.** Composition according to one or other of claims 10 and 11, for use as a medicament.

**13.** Composition according to claim 12, for use in the treatment and/or prevention of type 2 diabetes and/or complications thereof, and/or diseases able to be treated and/or prevented by increasing the level of HDL cholesterol and/or by reducing or preventing atheroma plaques, and/or diseases of the central nervous system.

**14.** Use of a methyl cyclodextrin as defined in any one of claims 1 to 6, or of a methyl cyclodextrin obtained according to the method as defined in any one of claims 7 to 9, for solubilising lipophilic compounds, or ones carrying at least one lipophilic group, and/or for improving the chemical stability thereof, and/or for improving the delivery thereof at and through the biological membranes, and/or for increasing the physical stability thereof, and/or for converting them from a liquid form to a powdery form, and/or for preventing interactions with other compounds, and/or for reducing local irritation after a topical or oral administration of these lipophilic compounds or ones carrying lipophilic groups, and/or for preventing absorption thereof in certain tissues such as the skin, and/or for obtaining a prolonged release of these compounds, and/or for masking the taste thereof, and/or for modifying the bioavailability thereof.

**Figure 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015087016 A1
- US 7259153 B2
- US 6602860 B1
- US 5935941 A
- US 9935941 A

**Littérature non-brevet citée dans la description**

- **KISS, T. ; FENYVESI, F. et al.** *Eur. J. Pharm. Sci.,* 2010, vol. 40, 376-380
- Cyclodextrines hydrophiles : caractérisation et étude de leurs propriétés énantiosélective et complexante. Utilisation de la chromatographie en phase liquide et de la spectrométrie de masse. **JACQUET ROMAIN.** Thèse de Chimie et physicochimie des composés d'intérêt biologique. Université d'Orléans, 2006, 59-83